# EUROPÄISCHE PATENTSCHRIFT

(11) **EP 0 812 317 B1**
(45) Veröffentlichungstag und Bekanntmachung des Hinweises auf die Patenterteilung: **08.09.1999**
(21) Anmeldenummer: 96916056.3
(22) Anmeldetag: 13.05.1996
(51) Int. Cl.: C07D 249/12, C07D 271/07, C07D 271/113, C07D 285/13, C07D 401/04, C07D 403/04, C07D 413/04, C07D 417/04, A01N 43/653, A01N 43/82

(54) **PESTIZIDE UND FUNGIZIDE 2-[2-(HETARYLOXYMETHYLEN)PHENYL]-CROTONATE**
2-[2-(HETARYL OXYMETHYLENE)PHENYL] CROTONATES USED AS PESTICIDES AND FUNGICIDES
2-[2-(HETARYLOXYMETHYLENE)PHENYL]-CROTONATES UTILISES COMME PESTICIDES ET FONGICIDES

(30) Priorität: 24.05.1995 DE 19519040
(43) Veröffentlichungstag der Anmeldung: 17.12.1997
(73) Patentinhaber: BASF AKTIENGESELLSCHAFT, 67056 Ludwigshafen (DE)
(72) Erfinder: GRAMMENOS, Wassilios, D-67063 Ludwigshafen (DE); MÜLLER, Bernd, D-67227 Frankenthal (DE); SAUTER, Hubert, D-68167 Mannheim (DE); OBERDORF, Klaus, D-69117 Heidelberg (DE); KÖNIG, Hartmann, D-69115 Heidelberg (DE); GÖTZ, Norbert, D-67547 Worms (DE); RACK, Michael, D-69123 Heidelberg (DE); LORENZ, Gisela, D-67434 Hambach (DE); AMMERMANN, Eberhard, D-64646 Heppenheim (DE); STRATHMANN, Siegfried, D-67117 Limburgerhof (DE)
(86) Internationale Anmeldenummer: EP9602043
(87) Internationale Veröffentlichungsnummer: WO9637480

(56) Entgegenhaltungen:
- EP-A- 0 280 185
- EP-A- 0 513 580

## Beschreibung

Die vorliegende Erfindung betrifft Verbindungen der allgemeinen Formel I 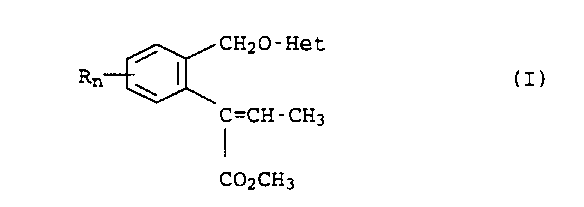 in der der Index und die Substituenten die folgende Bedeutung haben:
- R: Cyano, Halogen, C₁-C₄-Alkyl oder C₁-C₄-Alkoxy;
- n: 0, 1 oder 2, wobei die Substituenten R verschieden sein können, wenn n für 2 steht;
- Het: ein 5-gliedriger Heteroaromat enthaltend drei Stickstoffatome oder zwei Stickstoffatome und ein Sauerstoff- oder Schwefelatom, welcher einen ggf. substituierten Phenylring oder einen ggf. substituierten 6-gliedrigen Heteroaromaten, der seinerseits ein bis vier Stickstoffatome enthält, trägt.

Außerdem betrifft die Erfindung Verfahren zur Herstellung dieser Verbindungen, sie enthaltende Mittel und deren Verwendung zur Bekämpfung von Schädlingen oder Schadpilzen.

Aus der Literatur sind Hetaryloxymethylenphenylcrotonate bekannt, bei denen der Heteroaromat in der Position von Het eine Phenylgruppe tragen kann (EP-A 513 580).

Der Erfindung lagen demgegenüber Verbindungen mit verbesserten Eigenschaften als Aufgabe zugrunde.

Demgemäß wurden die eingangs definierten Verbindungen I gefunden. Außerdem wurden Verfahren zu ihrer Herstellung, sie enthaltende Mittel und deren Verwendung zur Bekämpfung von Schädlingen oder Schadpilzen gefunden.

Die Herstellung der Verbindungen I erfolgt in Analogie zu den in der eingangs zitierten Literatur beschriebenen Methoden dadurch, daß man eine Verbindung der Formel II in einem inerten organischen Lösungsmittel in Gegenwart einer Base mit einer Benzylverbindung der Formel III umsetzt. 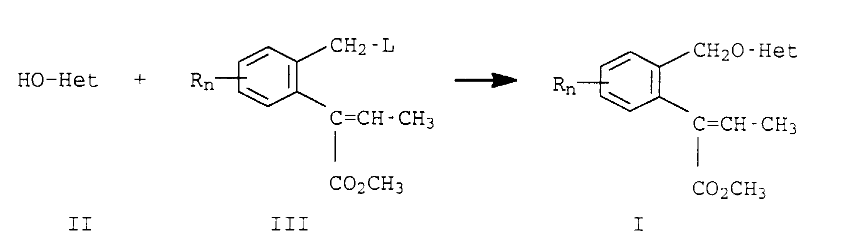 L in der Formel III steht für eine nucleophil austauschbare Abgangsgruppe wie Halogen (z.B. Fluor, Chlor, Brom und Iod, insbesondere Chlor, Brom und Iod) oder einen Sulfonylrest (z.B. Methylsulfonyl, Trifluormethylsulfonyl, Phenylsulfonyl und Methylphenylsulfonyl).

Diese Umsetzung erfolgt üblicherweise bei Temperaturen von 0°C bis 120°C, vorzugsweise 20°C bis 90°C.

Geeignete Lösungsmittel sind Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Tetrahydrofuran, Acetonitril, Aceton und Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, Alkalimetall- und Erdalkalimetallalkoholate wie Natrium-methanolat, Natriumethanolat, Kaliumethanolat, Kalium- tert.-Butanolat und Dimethoxymagnesium außerdem organische Basen,
z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydrid, Kaliumcarbonat und Natriummethanolat. Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, II in einem Über- oder Unterschuß bezogen auf III einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Herstellung der Verbindungen I benötigten Ausgangsstoffe II sind in der Literatur bekannt
[Hydroxytriazol: DE-A 24 17 970; DE-A 22 60 015; Synthesis 1987, 986; J. Med. Chem. 33, 2772 (1990); Chem. Ber. 56, 1794 (1923); DE-A 21 50 169; DE-A 22 00 436; US-A 4,433,148; Hydroxyoxadiazol: J. Am. Pharm. Assoc. 67, 799 (1958); J. Org. Chem. 27, 3472 (1962); J. Heterocycl. Chem. 19, 541 (1982); Chem. Ber. 18, 2467 (1885); Chem. Ber. 18, 2456 (1885); Chem. Ber. 19, 1475 (1886); Chem. Ber. 19, 1481 (1886); Comp. Rend. 26(1), 174 (1965); DE-A 22 12 797; Hydroxythiadiazole: Monatsh. d. Chem. 113(6-7), 793 (1982); EP-A 455 356; EP-A 389 901; EP-A 307 142; DE-A 40 31 158] oder können gemäß der zitierten Literatur hergestellt werden.

Die Herstellung der Verbindungen III ist in der EP-A 513 580 beschrieben.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: ein gesättigter, geradkettiger oder verzweigter Kohlenwasserstoffrest mit 1 bis 6 Kohlenstoffatomen, z.B. Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl: eine geradkettige oder verzweigte Alkylgruppe mit 1 bis 6 Kohlenstoffatomen (wie vorstehend genannt), wobei in dieser Gruppe die Wasserstoffatome teilweise oder vollständig durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: eine geradkettige oder verzweigte Alkylgruppe mit 1 bis 6 Kohlenstoffatomen (wie vorstehend genannt), welche über ein Sauerstoffatom (-O-) an das Gerüst gebunden ist;
Halogenalkoxy: eine geradkettige oder verzweigte Halogenalkylgruppe mit 1 bis 6 Kohlenstoffatomen (wie vorstehend genannt), welche über ein Sauerstoffatom (-O-) an das Gerüst gebunden ist;
Alkylamino: eine geradkettige oder verzweigte Alkylgruppe mit 1 bis 6 Kohlenstoffatomen (wie vorstehend genannt), welche über eine Aminogruppe (-NH-) an das Gerüst gebunden ist;
Dialkylamino: eine Aminogruppe, welche zwei voneinander unabhängige, geradkettige oder verzweigte Alkylgruppen mit jeweils 1 bis 6 Kohlenstoffatomen (wie vorstehend genannt) trägt;
Alkoxycarbonyl: eine geradkettige oder verzweigte Alkylgruppe mit 1 bis 6 Kohlenstoffatomen (wie vorstehend genannt), welche über ein Oxycarbonylgruppe (-O-CO-) an das Gerüst gebunden ist;
5-gliedriger Heteroaromat: ein 5-gliedriger Heteroaromat mit drei Stickstoffatomen als Ringgliedern oder ein 5-gliedriger Heteroaromat mit zwei Stickstoffatomen und einem Sauerstoff- oder Schwefelatom als Ringgliedern, z.B. Triazol, Oxadiazol und Thiadiazol;
6-gliedriger Heteroaromat: ein 6-gliedriger Heteroaromat mit ein bis vier Stickstoffatomen als Ringgliedern, z.B. Pyridin, Pyridazin, Pyrimidin, Pyrazin, Triazin und Tetrazin.

Insbesondere werden Verbindungen I bevorzugt, in denen n für 0 oder 1 steht.

Außerdem werden Verbindungen I besonders bevorzugt, in denen n für 1 steht und R Cyano, Fluor, Chlor, Methyl oder Methoxy bedeutet.

Gleichermaßen besonders bevorzugt sind Verbindungen I, in denen Het für eine Gruppe der Formeln II.1 bis II.5 steht: 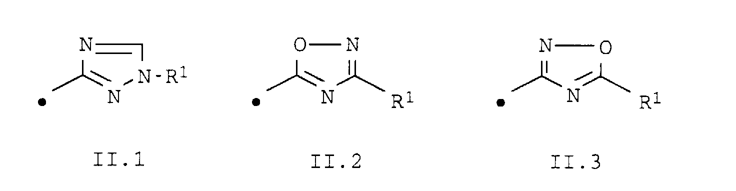 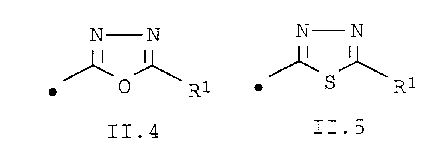 Bevorzugt werden Verbindungen I, in denen R¹ die folgende Bedeutung hat:

Phenyl oder ein 6-gliedriger Heteroaromat enthaltend ein bis drei Stickstoffatome, wobei diese Reste partiell oder vollständig halogeniert sein können und/oder ein bis drei der folgenden Gruppen tragen können: C₁-C₆-Alkyl, C₁-C₆-Halogenalkyl, C₁-C₆-Alkoxy, C₁-C₆-Halogenalkoxy, C₁-C₆-Alkylamino, Di-C₁-C₆-alkylamino und C₁-C₆-Alkoxycarbonyl.

Insbesondere werden Verbindungen I bevorzugt, in denen R¹ ggf. subst. Phenyl bedeutet.

Gleichermaßen besonders bevorzugt werden Verbindungen I, in denen R¹ Phenyl bedeutet, welches partiell oder vollständig halogeniert sein kann und/oder ein bis fünf der folgenden Reste tragen kann: C₁-C₄-Alkyl, C₁-C₄-Halogenalkyl, C₁-C₄-Alkyloxy, C₁-C₄-Halogenalkyloxy, C₁-C₄-Alkylamino, Di-C₁-C₄-Alkylamino und C₁-C₄-Alkoxycarbonyl.

Insbesondere werden Verbindungen I bevorzugt, in denen Het einen der folgenden Reste trägt: ggf. subst. Pyridin, Pyrimidin, Pyrazin und Triazin.

Außerdem werden Verbindungen I besonders bevorzugt, in denen der 6-gliedrige Heteroaromat an Het partiell oder vollständig halogeniert ist und/oder ein bis drei der folgenden Reste trägt:

C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-alkylamino und C₁-C₄-Alkoxycarbonyl.

Insbesondere bevorzugt sind Verbindungen I, in denen der 6-gliedrige Heteroaromat an Het ein bis drei der folgenden Reste tragen kann: Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-alkylamino und C₁-C₄-Alkoxycarbonyl.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet (unabhängig von der Kombination, in der sie genannt sind) eine besonders bevorzugte Ausgestaltung des betreffenden Substituenten dar.

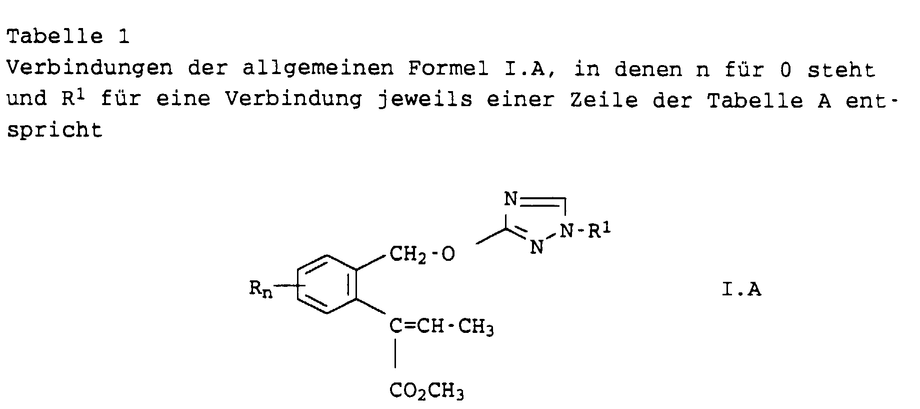

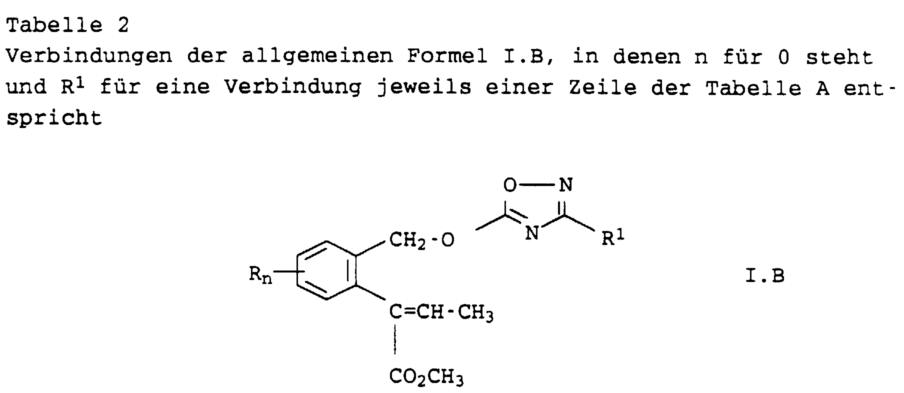

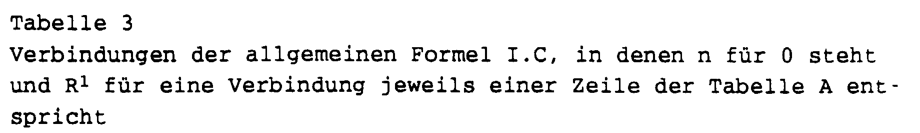

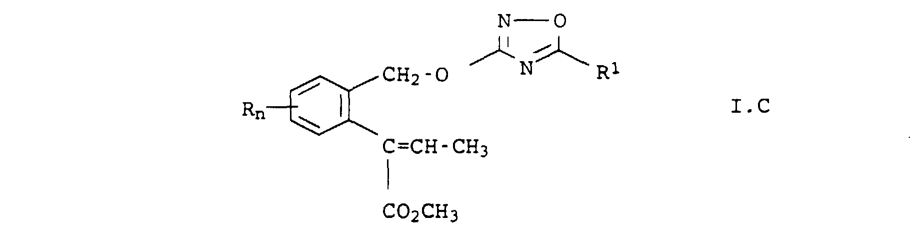

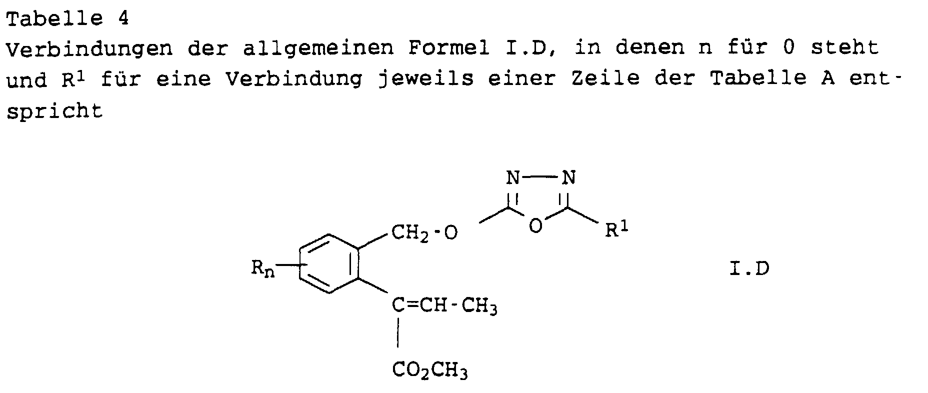

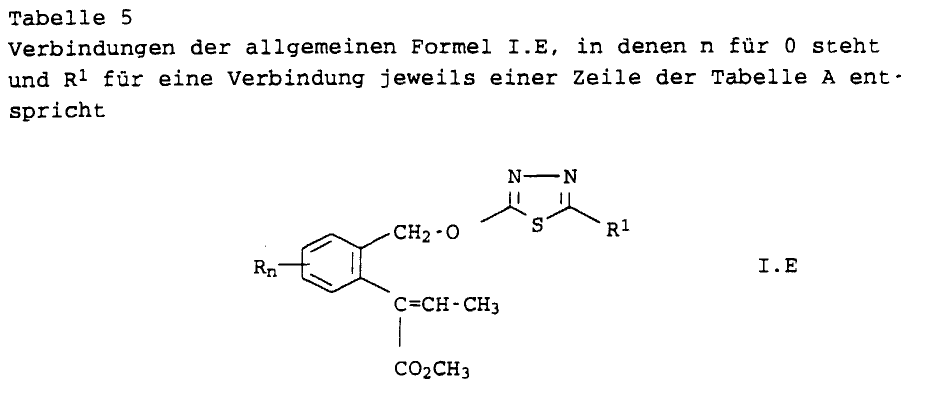

**Tabelle A**

| R¹ | R¹ | R¹ |
|---|---|---|
| C₆H₅ | 2-CH₃-C₆H₄ | 2-F, 4-CH₃-C₆H₃ |
| 2-Cl-C₆H₄ | 3-CH₃-C₆H₄ | 4-F, 2-CH₃-C₆H₃ |
| 3-Cl-C₆H₄ | 4-CH₃-C₆H₄ | 5-Cl, 2-CH₃-C₆H₃ |
| 4-Cl-C₆H₄ | 2,3-(CH₃)₂-C₆H₃ | 4-C₂H₅-C₆H₄ |
| 2-F-C₆H₄ | 2,4-(CH₃)₂-C₆H₃ | 4-CH(CH₃)₂-C₆H₄ |
| 3-F-C₆H₄ | 2,5-(CH₃)₂-C₆H₃ | 4-C(CH₃)₃-C₆H₄ |
| 4-F-C₆H₄ | 2,6-(CH₃)₂-C₆H₃ | 4-OC₂H₅-C₆H₄ |
| 2-Br-C₆H₄ | 3,4-(CH₃)₂-C₆H₃ | 4-OCH(CH₃)₂-C₆H₄ |
| 3-Br-C₆H₄ | 3,5-(CH₃)₂-C₆H₃ | 4-OC(CH₃)₃-C₆H₄ |
| 4-Br-C₆H₄ | 2-CF₃-C₆H₄ | pyridin-2-yl |
| 2,3-Cl₂-C₆H₃ | 3-CF₃-C₆H₄ | pyridin-3-yl |
| 2,4-Cl₂-C₆H₃ | 4-CF₃-C₆H₄ | pyridin-4-yl |
| 2,5-Cl₂-C₆H₃ | 2,3-(CF₃)₂-C₆H₃ | 6-Cl-pyridin-2-yl |
| 2,6-Cl₂-C₆H₃ | 2,4-(CF₃)₂-C₆H₃ | 5-Cl-pyridin-2-yl |
| 3,4-Cl₂-C₆H₃ | 2,5-(CF₃)₂-C₆H₃ | 6-CH₃-pyridin-2-yl |
| 3,5-Cl₂-C₆H₃ | 2,6-(CF₃)₂-C₆H₃ | 5-CH₃-pyridin-2-yl |
| 2,3-F₂-C₆H₃ | 3,4-(CF₃)₂-C₆H₃ | 6-C₂H₅-pyridin-2-yl |
| 2,4-F₂-C₆H₃ | 3,5-(CF₃)₂-C₆H₃ | 6-CH(CH₃)₂-pyridin-2-yl |
| 2,5-F₂-C₆H₃ | 2-OCH₃-C₆H₄ | 6-C(CH₃)₃-pyridin-2-yl |
| 2,6-F₂-C₆H₃ | 3-OCH₃-C₆H₄ | 6-CF₃-pyridin-2-yl |
| 3,4-F₂-C₆H₃ | 4-OCH₃-C₆H₄ | 5-CF₃-pyridin-2-yl |
| 3,5-F₂-C₆H₃ | 2,3-(OCH₃)₂-C₆H₃ | 4-CH₃-pyridin-2-yl |
| 2,3-Br₂-C₆H₃ | 2,4-(OCH₃)₂-C₆H₃ | 6-F-pyridin-2-yl |
| 2,4-Br₂-C₆H₃ | 2,5-(OCH₃)₂-C₆H₃ | 5-F-pyridin-2-yl |
| 2,5-Br₂-C₆H₃ | 2,6-(OCH₃)₂-C₆H₃ | 4-F-pyridin-2-yl |
| 2,6-Br₂-C₆H₃ | 3,4-(OCH₃)₂-C₆H₃ | pyrimidin-2-yl |
| 3,4-Br₂-C₆H₃ | 3,5-(OCH₃)₂-C₆H₃ | pyrimidin-4-yl |
| 3,5-Br₂-C₆H₃ | 2,3,5-(CH₃)₃-C₆H₂ | pyrimidin-5-yl |
| 2,3,5-Cl₃-C₆H₂ | 2,3,5-(CF₃)₃-C₆H₂ | 4-CH₃-pyrimidin-2-yl |
| 2,3,5-Br₃-C₆H₂ | 2,3,5-(OCH₃)₃-C₆H₂ | 4,6-(CH₃)₂-pyrimidin-2-yl |
| 2,3,5-F₃-C₆H₂ | 2-F, 5-CH₃-C₆H₃ | 4,6-(OCH₃)₂-pyrimidin-2-yl |
| 2-F, 4-Cl-C₆H₃ | 5-F, 2-CH₃-C₆H₃ | 6-CH₃-pyrimidin-4-yl |
| 4-F, 2-Cl-C₆H₃ | 2-Cl, 4-CH₃-C₆H₃ | pyridazin-2-yl |
| 4-Cl, 2-CH₃-C₆H₃ | 2-Cl, 5-CH₃-C₆H₃ | triazin-2-yl |

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, l-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calcium-salz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile einer erfindungsgemäßen Verbindung werden in einer Mischung gelost, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII.20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1

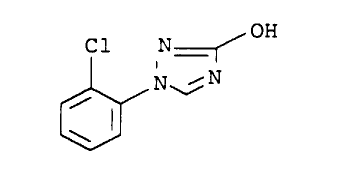

Zu 50 g 2-Chlorphenylhydrazin-hydrochlorid in 200 ml Wasser tropft man 29 g Kaliumcyanat in 100 ml Wasser. Anschließend läßt man über Nacht bei RT rühren, saugt die ausgefallenen Kristalle ab, wäscht sie mit Wasser und trocknet sie. Man erhält 49,32 g (Schmp. 178,7°C), die ohne weitere Reinigung mit 100 ml Triethylorthoformiat versetzt werden. Anschließend rührt man bei 120°C und destilliert das dabei gebildete Ethanol ab. Das ausgefallene Produkt wird abgesaugt mit Methylenchlorid und einmal mit Methyl-tert-butyl-ether nachgewaschen und getrocknet. Man erhält 33,7 g Produkt in Form farbloser Kristalle, die ohne weitere Reinigung bei Beispiel 2 eingesetzt werden.

### Beispiel 2

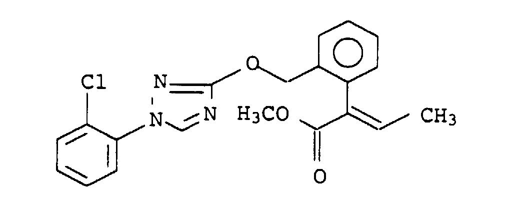

25,4 g 3-Hydroxy-[2-chlorphenyl)-triazol aus Beispiel 1 werden in 900 ml DMF gelöst und mit 62,9 g Kaliumcarbonat versetzt. Anschließend gibt man 100 mg Kaliumjodid dazu, und tropft bei RT die Lösung von 35 g α-[Brommethyl-phenyl)-β-methylacrylsäuremethylester in 500 ml DMF zu. Man rührt 5 Stunden bei 45°C und fällt das Produkt durch Zugabe von Eiswasser aus. Dann wird in Essigester aufgenommen, mit Wasser gewaschen, über Natriumsulfat getrocknet und eingeengt. Man erhält 46,3 g Produkt, als dickflüssiges Öl.
¹H-NMR(CDCl₃) : δ = 8,7-7,1 (m, 8H) S,2 (S,2H), 3,7 (S,3H), 1,65 (d, 3H).

### Beispiel 3

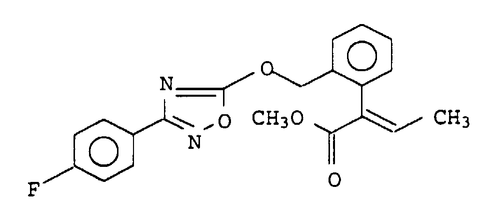

1 g 5-Hydroxy-3-(4-fluorphenyl)-oxadiazol werden zusammen mit 50 ml n-Hexan, 1,5 g Silbercarbonat und 1,5 g α-(2-Brommethylphenyl)-β-methylacrylsäuremethylester 10 Stunden unter Rückfluß gekocht. Dann gibt man noch 1 g Silbercarbonat und 50 ml n-Hexan und kocht weitere 10 Stunden unter Rückfluß. Zur Aufarbeitung wird abfiltriert, mit n-Hexan nachgewaschen, und die n-Hexan-Phase mit Wasser gewaschen, über Natriumsulfat getrocknet und eingeengt. Man erhält 0,7 g Produkt, welches zur weiteren Reinigung über Kieselgel mit Essigester/n-Heptan (1:1) chromatographiert werden kann.
¹H-NMR(CDCl₃): δ = 8,0 (m, 2H) 7,6 (m,1H), 7,5-7,1 (m, 5H) 5,4 (S,2H), 3,7 (S,3H) und 1,65 (d,3H).

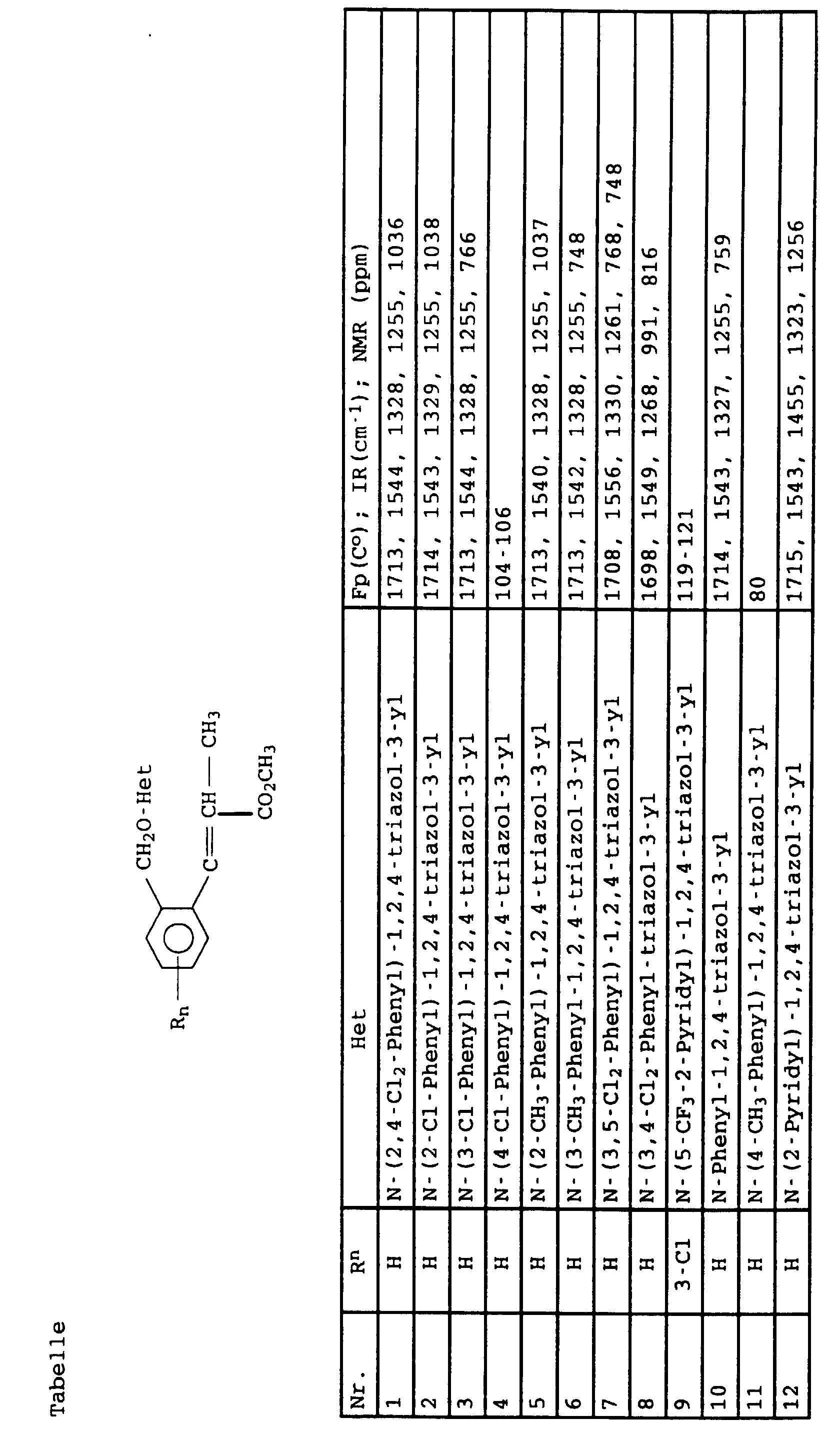

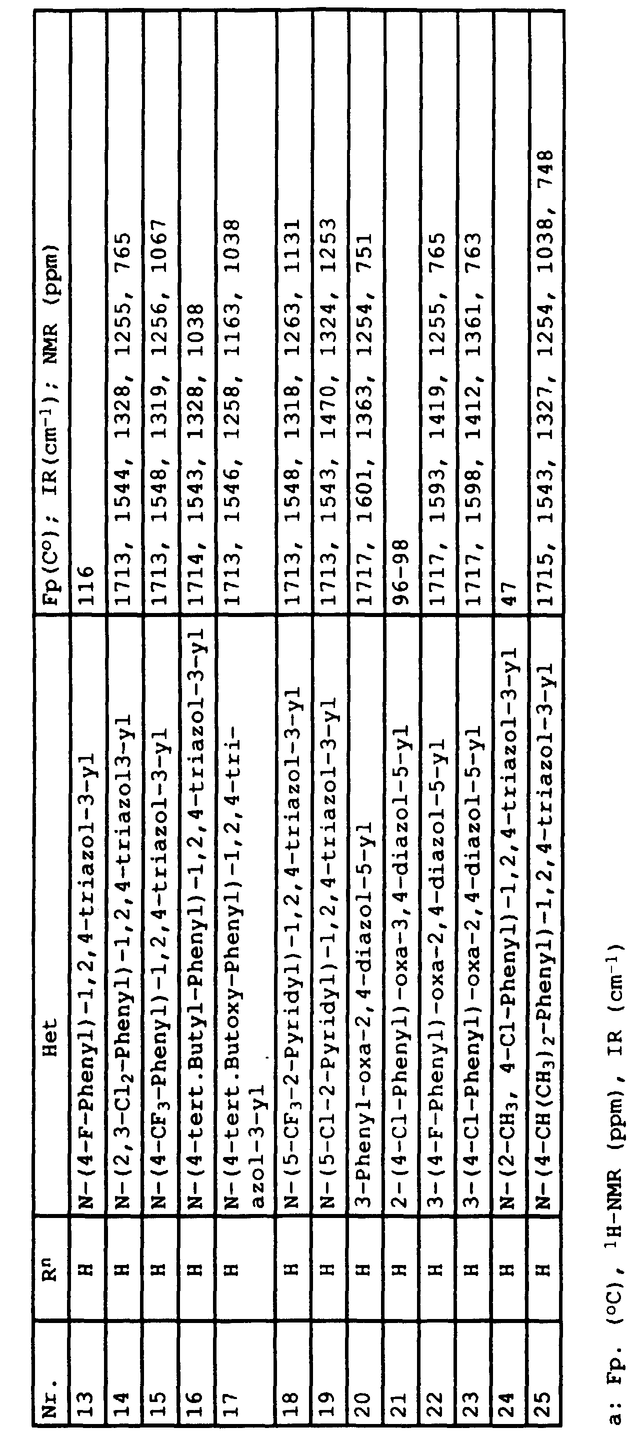

### Beispiele für die Wirkung gegen Schadpilze

Die fungizide Wirkung der Verbindungen der allgemeinen Formel I ließ sich durch die folgenden Versuche zeigen:

Die Wirkstoffe wurden als 20%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Plasmopara viticola (Rebenperonospora)

Topfreben (Sorte: "Müller Thurgau") wurden mit der Wirkstoffaufbereitung tropfnaß gespritzt (Aufwandmenge 16 ppm). Nach 8 Tagen wurden die Pflanzen mit einer Zoosporenaufschwemmung des Pilzes *Plasmopara viticola* besprüht und 5 Tage bei 20-30°C bei hoher Luftfeuchtigkeit bewahrt. Vor der Beurteilung wurden die Pflanzen danach für 16 h bei hoher Luftfeuchtigkeit bewahrt. Die Auswertung erfolgte visuell.

In diesem Test zeigten die mit den erfindungsgemäßen Verbindungen Nrs. 1, 2, 4, 6, 7, 10, 11 und 13 behandelten Pflanzen einen Befall von 10% und weniger während die mit den bekannten Wirkstoffen A bzw. B (EP-A 280 185, Beispiel Nr. 124 bzw. Nr. 127) behandelten Pflanzen zu 40% befallen waren. Die unbehandelten (Kontroll-) Pflanzen waren zu 70% befallen.

In einem entsprechenden Test zeigten die mit den erfindungsgemäßen Verbindungen 14, 15, 16, 18, 22, 23, 24 und 25 behandelten Pflanzen einen Befall von 15 % und weniger, während die unbehandelten (Kontroll-)Pflanzen zu 70 % befallen waren.

### Wirkung gegen Puccinia recondita (Weizenbraunrost)

Blätter von Weizensämlingen (Sorte "Kanzler") wurden mit Sporen des Braunrosts *(Puccinia recondita)* bestäubt. Die so behandelten Pflanzen wurden 24 h bei 20-22°C und einer relativen Luftfeuchtigkeit von 90-95% inkubiert und anschließend mit der wäßrigen Wirkstoffaufbereitung behandelt (Aufwandmenge: 63 ppm). Nach weiteren 8 Tagen bei 20-22°C und 65-70% relativer Luftfeuchtigkeit wurde das Ausmaß der Pilzentwicklung ermittlelt. Die Auswertung erfolgte visuell.

In diesem Test zeigten die mit den erfindungsgemäßen Verbindungen Nrs. 1, 2, 4, 6 und 9 behandelten Pflanzen einen Befall von 5% und weniger während die mit den bekannten Wirkstoffen A bzw. B (EP-A 280 185, Beispiel Nr. 124 bzw. Nr. 127) behandelten Pflanzen zu 60% bzw. 25% befallen waren. Die unbehandelten (Kontroll-) Pflanzen waren zu 75% befallen.

In einem entsprechenden Test zeigten die mit den erfindungsgemäßen Verbindungen 15, 16, 18, 24 und 25 behandelten Pflanzen einen Befall von 15 % und weniger, während die unbehandelten (Kontroll-)Pflanzen zu 75 % befallen waren.

### Beispiele für die Wirkung gegen tierische Schädlinge

Die Wirkung der Verbindungen der allgemeinen Formel I gegen tierische Schädlinge ließ sich durch folgende Versuche zeigen: Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. Verbindungen der allgemeinen Formel I 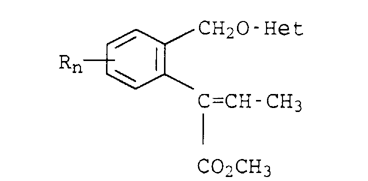 in der der Index und die Substituenten die folgende Bedeutung haben:
R Cyano, Halogen, C₁-C₄-Alkyl oder C₁-C₄-Alkoxy;
n 0, 1 oder 2, wobei die Substituenten R verschieden sein können, wenn n für 2 steht;
Het ein 5-gliedriger Heteroaromat enthaltend drei Stickstoffatome oder zwei Stickstoffatome und ein Sauerstoff- oder Schwefelatom, welcher einen ggf. substituierten Penylring oder einen ggf. substituierten 6-gliedrigen Heteroaromaten, der seinerseits ein bis vier Stickstoffatome enthält, trägt.

2. Verbindungen der allgemeinen Formel I gemäß Anspruch 1, in der Het für einen 5-gliedrigen Heteroaromaten der Formeln II.1 bis II.5 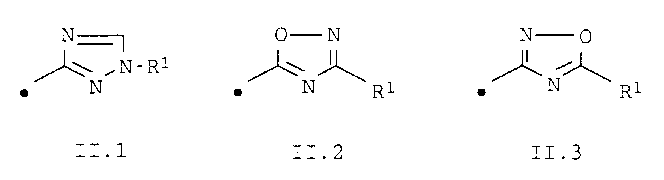 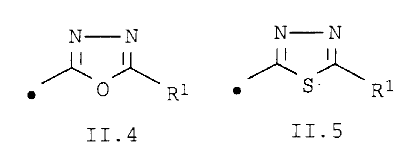 steht, wobei die mit • gekennzeichnete Bindung die Bindung zum Sauerstoffatom repräsentiert; und
R¹ für einen ggf. substituierten 6-gliedrigen Heteroaromaten, der seinerseits ein bis vier Stickstoffatome enthält, steht.

3. Verbindungen der allgemeinen Formel I gemäß Anspruch 1, in der Het für einen 5-gliedrigen Heteroaromaten der Formeln II.1 bis II.5 gemäß Anspruch 2 steht und R¹ die folgende Bedeutung hat:
Phenyl oder ein 6-gliedriger Heteroaromat enthaltend ein bis drei Stickstoffatome, wobei diese Reste partiell oder vollständig halogeniert sein können und/oder ein bis drei der folgenden Gruppen tragen können: C₁-C₆-Alkyl, C₁-C₆-Halogenalkyl, C₁-C₆-Alkoxy, C₁-C₆-Halogenalkoxy, C₁-C₆-Alkylamino, Di-C₁-C₆-alkylamino und C₁-C₆-Alkoxycarbonyl.

4. Verfahren zur Herstellung der Verbindungen I gemäß Anspruch 1, dadurch gekennzeichnet, daß man eine Verbindung der Formel II
HO-Het II
in an sich bekannter Weise in einem inerten organischen Lösungsmittel in Gegenwart einer Base mit einer Benzylverbindung der Formel III, 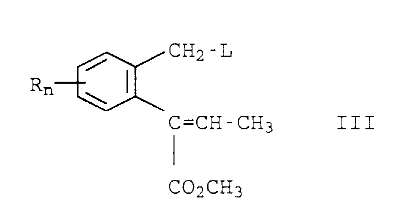 in der L für eine nucleophil austauschbare Abgangsgruppe steht, umsetzt.

5. Zur Bekämpfung von Schädlingen oder Schadpilzen geeignetes Mittel, enthaltend einen festen oder flüssigen Trägerstoff und eine Verbindung der allgemeinen Formel I gemäß Anspruch 1.

6. Verwendung der Verbindungen I gemäß Anspruch 1 zur Herstellung eines zur Bekämpfung von Schädlingen oder Schadpilzen geeigneten Mittels.

7. Verfahren zur Bekämpfung von Schadpilzen, dadurch gekennzeichnet, daß man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

8. Verfahren zur Bekämpfung von Schädlingen, dadurch gekennzeichnet, daß man die Schädlingen oder die von ihnen zu schützenden Materialien, Pflanzen, Boden oder Saatgüter mit II.1 einer wirksamen Menge einer Verbindung der allgemeinen Formel I gemäß Anspruch 1 behandelt.

## Claims

1. A compound of the general formula I 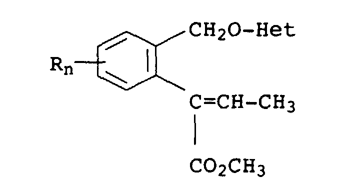 where the index and the substituents have the following meanings:
R is cyano, halogen, C₁-C₄-alkyl or C₁-C₄-alkoxy;
n is 0, 1 or 2, it being possible for the substituents R to differ from each other if n is 2;
Het is a 5-membered heteroaromatic ring having three nitrogen atoms or two nitrogen atoms and one oxygen or sulfur atom and having attached to it an unsubstituted or substituted phenyl ring or an unsubstituted or substituted 6-membered heteroaromatic ring which, in turn, has one to four nitrogen atoms.

2. A compound of the general formula I as claimed in claim 1 where Het is a 5-membered heteroaromatic ring of the formulae II.1 to II.5 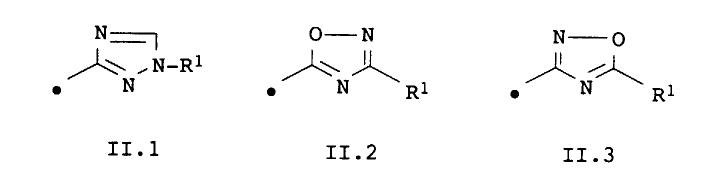 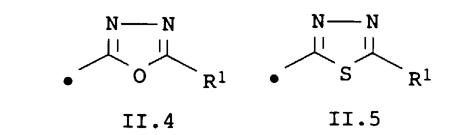 where the bond designated by • represents the bond to the oxygen atom; and
R¹ represents an unsubstituted or substituted 6-membered heteroaromatic ring which, in turn, has one to four nitrogen atoms.

3. A compound of the general formula I as claimed in claim 1 where Het is a 5-membered heteroaromatic ring of the formulae II.1 to II.5 as claimed in claim 2 and R¹ has the following meanings:
phenyl or a 6-membered heteroaromatic ring which has one to three nitrogen atoms, it being possible for these radicals to be partially or fully halogenated and/or to have attached to them one to three of the following groups: C₁-C₆-alkyl, C₁-C₆-haloalkyl, C₁-C₆-alkoxy, C₁-C₆-haloalkoxy, C₁-C₆-alkylamino, di-C₁-C₆-alkylamino and C₁-C₆-alkoxycarbonyl.

4. A process for the preparation of the compounds I as claimed in claim 1, which comprises reacting a compound of the formula II
HO-Het II
in a manner known per se with a benzyl compound of the formula III 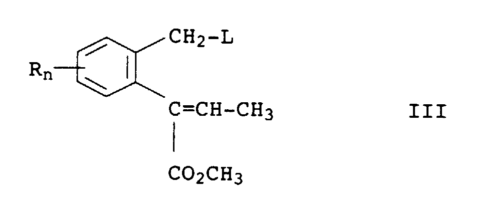 where L is a nucleophilically exchangeable leaving group, in an inert organic solvent in the presence of a base.

5. A composition which is suitable for controlling animal or fungal pests which comprises a solid or liquid carrier and a compound of the general formula I as claimed in claim 1.

6. The use of the compounds I as claimed in claim 1 for the preparation of a composition which is suitable for controlling animal or fungal pests.

7. A method of controlling fungal pests, which comprises treating the fungi or the materials, plants, soil or seed to be protected against fungal infection with an effective amount of a compound of the general formula I as claimed in claim 1.

8. A method of controlling animal pests, which comprises treating the animal pests or the materials, plants, soil or seed to be protected against them with an effective amount of a compound of the general formula I as claimed in claim 1.

## Revendications

1. Composés de formule générale I 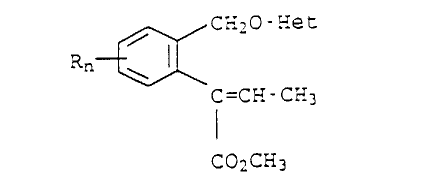 dans laquelle l'indice et les symboles ont les significations suivantes :
R : un groupe cyano, un halogène, un groupe alkyle en C1-C4 ou alcoxy en C1-C4 ;
n : 0, 1 ou 2, les substituants R pouvant être différents lorsque n est égal à 2 ;
Het : un radical hétéroaromatique à cinq chaînons contenant trois atomes d'azote ou deux atomes d'azote et un atome d'oxygène ou de soufre, qui porte un noyau phényle éventuellement substitué ou un cycle hétéroaromatique à six chaînons contenant lui-même un à quatre atomes d'azote et qui peut être substitué.

2. Composés de formule générale I selon l'invention 1, dans laquelle Het représente un radical hétéroaromatique à cinq chaînons répondant à l'une des formules II.1 à II.5 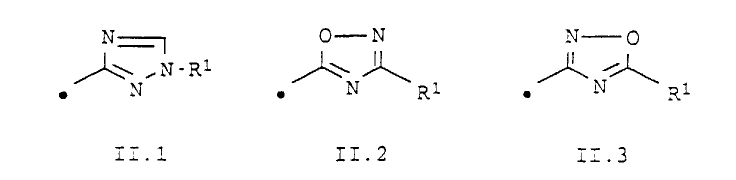 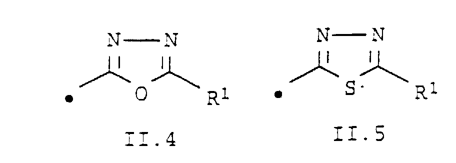 dans lesquelles la liaison signalée par . représente la liaison à l'atome d'oxygène ; et
R¹ représente un groupe hétéroaromatique à six chaînons, contenant lui-même un à quatre atomes d'azote et qui peut être substitué.

3. Composés de formule générale I selon la revendication 1, dans laquelle Het représente un groupe hétéroaromatique à cinq chaînons répondant à l'une des formules II.1 à II.5 de la revendication 2 et R¹ a la signification suivante :
un groupe phényle ou un groupe hétéroaromatique à six chaînons contenant un à trois atomes d'azote, ces groupes pouvant être partiellement ou totalement halogénés et/ou pouvant porter un à trois des substituants suivants : alkyle en C1-C6, halogénoalkyle en C1-C6, alcoxy en C1-C6, halogénoalcoxy en C1-C6, alkylamino en C1-C6, di-(alkyle en C1-C6)amino et (alcoxy en C1-C6)carbonyle.

4. Procédé de préparation des composés I de la revendication 1, caractérisé par le fait que l'on fait réagir un composé de formule II
HO-Het II
de manière connue en soi, dans un solvant organique inerte et en présence d'une base, avec un dérivé benzylique de formule III 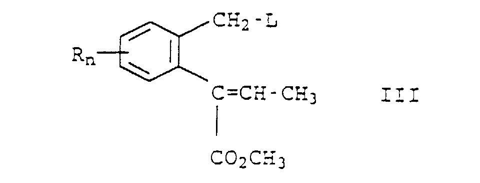 dans laquelle L représente un groupe éliminable par échange nucléophile.

5. Produit approprié à la lutte contre les parasites ou les mycètes nuisibles, contenant un véhicule solide ou liquide et un composé de formule générale I de la revendication 1.

6. Utilisation des composés de la revendication 1 pour la préparation d'un produit approprié à la lutte contre les parasites ou les mycètes nuisibles.

7. Procédé pour combattre les mycètes nuisibles, caractérisé par le fait que l'on traite les mycètes ou les matières, végétaux, sols ou semences qu'on veut protéger contre une attaque par les mycètes par une quantité efficace d'un composé de formule générale I de la revendication 1.

8. Procédé pour combattre les parasites, caractérisé par le fait que l'on traite les parasites ou les matières, végétaux, sols ou semences qu'on veut protéger contre les parasites, par une quantité efficace d'un composé de formule générale I de la revendication 1.
